# EUROPEAN PATENT SPECIFICATION

(11) **EP 0 563 127 B1**
(45) Date of publication and mention of the grant of the patent: **07.09.1994**
(21) Application number: 92901177.3
(22) Date of filing: 16.12.1991
(51) Int. Cl.: A61K 45/08, A61K 31/13, A61K 31/535, A61K 47/00

(54) **IMPROVED TREATMENT METHOD FOR CANCER**
VERBESSERTES BEHANDLUNGSVERFAHREN FÜR KREBS
PROCEDE AMELIORE DE TRAITEMENT DU CANCER

(30) Priority: 17.12.1990 US 627863; 07.06.1991 US 711975
(43) Date of publication of application: 06.10.1993
(73) Proprietor: UNIVERSITY OF MANITOBA, Winnipeg, Manitoba R3T 2N2 (CA)
(72) Inventor: BRANDES, Lorne, J., Winnipeg, Manitoba R3N 1A3 (CA)
(74) Representative: Burford, Anthony Frederick
(86) International application number: CA9100449
(87) International publication number: WO9211035

(56) References cited:
- EP-A- 0 153 160
- Dialog Information Services, File 155, MEDLINE; Dialog accession no. 05533338, Medline accession no. 85149338, Brandes LJ et al., "Evidence that the antiestrogen binding site is a hestimine or histamine-like receptor", Biochem Res Commun Jan 31 1985, 126 (2) p 905-910.
- Dialog Information Services, file 155, MEDLINE 66-92, May, Dialog accession no. 07418183, Medline accession no. 90325183, Brandes LJ et al., "Study of the in-vivo antioestrogenic action of N,N-diethyl-2-4-(phenylmethyl)phenoxyethanamine HC1 (DPPE), a novel intracellular histamine antagonist and antioestrogen binding site ligand", J Reprod Fertil May 1990, 89 (1) p 59-67.
- Dialog Information Services, File 159, Cancerlit 63-92 Mar, Dialog accession no. 00606997, 87629197, MEDL/87273175, Brandes LJ et al., "Histamine and growth: interaction of antiestrogen binding site ligaands with a novel histamine site that may be associated with calcium channels", Cancer Res; 47(15): 4025-31 1987.

## Description

The present invention relates to the improved treatment of cancer in animals, including humans, using chemotherapeutic agents.

One of the major chemotherapeutic treatments is that of malignant growth (cancer) in humans. The objective of chemotherapy is the total extermination of clonogenic tumor or malignant cells, with minimal damage to the patient. However, one of the major limitations of the chemotherapeutic approach for managing human cancer is the general inability of anticancer drugs to discriminate between normal and tumorous cells. Antineoplastic agents have the lowest therapeutic indicies of any class of drugs used in humans and hence produce significant and potentially life-threatening toxicities. Certain commonly-used anti-neoplastic agents have unique and acute toxicities for specific tissues. For example, the vinca alkaloids possess significant toxicity for nervous tissues, while adriamycin has specific toxicity for heart tissue and bleomycin has for lung tissue. In general, almost all members of the major categories of anti-neoplastic agents have considerable toxicities for normal cells of gastrointestinal, epidermal and myelopoietic tissues.

Generally, the dose-limiting consideration for chemical management of cancer in humans is the toxicity that anti-neoplastic agents have for the pluripotent stem cells of myelopoietic tissue. This toxicity arises from the fact that most anticancer drugs function preferentially against proliferating cells but with no significant capacity to discriminate between cycling normal and cycling tumor tissues.

Attempts have been made to confer specificity upon presently-available chemotherapeutic agents. In Anticancer Research 6:451 to 464 (1986), Robert C. Warrington describes certain in vitro and in vivo experiments demonstrating the achievement of improvements in both the specificity and efficacy of a number of commonly-used anticancer drugs by using these agents in combination with L-histidinol. L-histidinol is a structural analogue of the essential amino acid, histidine, in which the α-carboxyl group has been reduced to a primary alcohol. In the work presented by Warrington in this paper, L-histidinol was found to be effective at doses of approximately 1000 mg/kg of tissue administered five hours or more prior to the chemotherapeutic agent.

It now surprisingly has been found that, if an antagonist specific for a recently-discovered intracellular histamine receptor, designated H_{IC}, different from traditional histamine receptors classified as H₁, H₂ or H₃, is administered to a living animal having cancer, then the specificity and efficacy of chemotherapeutic agents for cancer cells is improved. By employing an antagonist specific to inhibit the binding of intracellular histamine, the improved effect is obtained at significantly lower dosage levels administered at a significantly shorter period of time prior to administration of the chemotherapeutic agent than is shown in the Warrington work referred to above.

The present invention is broadly applicable to the treatment of malignant cells in a living animal where the administration of chemotherapeutic agents normally adversely affects the normal cells in the animal. By first administering to the animal an antagonist specific for intracellular histamine in an amount sufficient to inhibit binding of intracellular histamine in normal cells at the intracellular histamine binding site, the specificity and efficacy of subsequently administered therapeutic agents on malignant cell is improved.

Accordingly, in one aspect of the present invention, there is provided the use of an antagonist specific for intracellular histamine for the manufacture of a medicament for the treatment of cancer cells in a living animal by administration to said animal in an amount sufficient to inhibit the binding of intracellular histamine in normal cells prior to administration of at least one chemotherapeutic agent for the cancer cells in an amount toxic to said cancer cells, to enhance the toxic effect on said cancer cells of said at least one chemotherapeutic agent while inhibiting any adverse effect of said at least one chemotherapeutic agent on said normal cells.

It has further been found that, if a patient being treated with a chemotherapeutic agent also is given an iv infusion of an antagonist specific for intracellular histamine over a period of from 24 to 72 hours after administration of the chemotherapeutic agent, then the side effects generally associated with chemotherapy, namely nausea, vomiting, anorexia and stomatitis, are at least ameliorated and often prevented.

Accordingly, in another aspect of the present invention, there is provided the use of an antagonist specific for intracellular histamine for the manufacture of a medicament for the treatment of cancer cells in a living animal by administration to said animal in an amount sufficient to inhibit the binding of intracellular histamine in normal cells over a period of at least twenty-four hours subsequent to administration of at least one chemotherapeutic agent for the cancer cells in an amount toxic to said cancer cells, to at least ameliorate the side effects of the administration of said at least one chemotherapeutic agent.

The invention further includes a kit useful in the latter procedure, comprising
(a) a first component consisting of an antagonist specific for intracellular histamine in a dosage amount sufficient to inhibit the binding of intracellular histamine in normal cells of the animal, and, separately,
(b) a second component consisting of at least one chemotherapeutic agent for the cancer cells in a dosage amount toxic to said cancer cells, and, separately:
(c) a third component consisting of an antagonist specific for intracellular histamine in a dosage amount sufficient to ameliorate the side effects of administration of the chemotherapeutic agent to the animal.

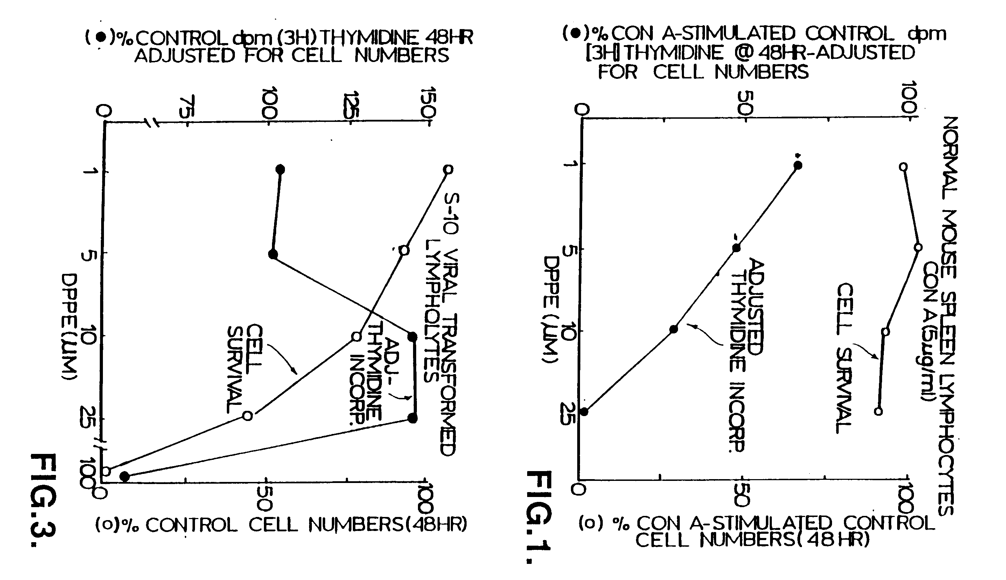

Figures 1 to 5 are graphical representations of test data generated in certain experiments set forth in the Examples below.

In the present invention, any compound which is an antagonist specific for the intracellular histamine receptor is useful and is administered in an amount sufficient to inhibit the binding of intracellular histamine at the intracellular binding site (H_{IC}) in normal cells.

Specific compounds which are useful in the present invention are diphenylmethanes of the formula: 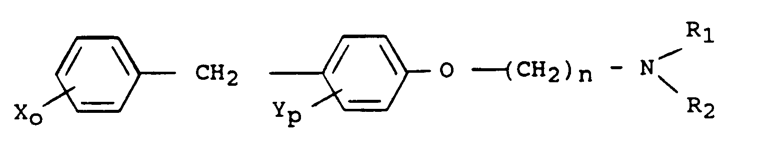
wherein X and Y are each chlorine or bromine, o and p are 0 or 1, R₁ and R₂ are each alkyl groups containing 1 to 3 carbon atoms or are joined together to form a hetero-ring with the nitrogen atom and n is 1, 2 or 3. Pharmaceutically-acceptable salts of the diphenylmethanes may be employed.

In one preferred embodiment, the group 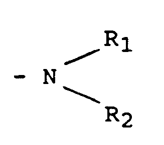
is a diethylamino group and, in another preferred embodiment, a morpholino group. o and p are usually 0 and n may be 2. In one particularly preferred embodiment, n is 2, o and p are each 0 and 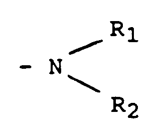
is a diethylamino group. This compound, namely N,N-diethyl-2-[4-(phenylmethyl)-phenoxy]ethanamine, in the form of its hydrochloride salt, is abbreviated herein as DPPE.

The compounds used herein are antagonists specific for intracellular histamine and specifically inhibit intracellular histamine binding at a site designated H_{IC}. L-histidinol used in the Warrington work referred to above, is not specific for intracellular histamine. Although some affinity for binding to H_{IC} is exhibited by L-histidinol, this compound also binds to other histamine binding sites. The present invention employs compounds which bind only to H_{IC}.

In the present invention, significantly-smaller quantities of the antagonist compound are used when compared to L-histidinol (typically 2 mg/kg vs. 1000 mg/kg) and the antagonist compound is administered in the present invention a much shorter period before the chemotherapeutic agent(s) when compared to L-histidinol (typically 20 to 30 minutes vs. 5 hours).

The antagonist compound employed in the present invention is administered to the patient in any convenient manner, such as by injection of a solution thereof in an aqueous pharmaceutically-acceptable vehicle.

The antagonist compound is administered to the patient before administration of at least one chemotherapeutic agent. The chemotherapeutic agent or more commonly a mixture of such agents may be administered in any convenient manner consistent with its normal manner of administration following conventional chemotherapeutic practice.

The administration of the antagonist compound to the patient prior to administration of the chemotherapeutic agent is necessary in order to permit the antagonist to inhibit the binding of intracellular histamine in normal cells and thereby, in effect, shut down proliferation of the normal cells.

The length of time prior to administration of the chemotherapeutic agent that the antagonist compound is administered depends on the antagonist compound, its mode of administration and the size of the patient. Generally, the antagonist compound is administered to the patient 15 to 90 minutes, preferably 30 to 60 minutes, prior to administration of the at least one therapeutic agent.

The quantity of antagonist compound administered to the patient should be at least sufficient to inhibit binding of intracellular histamine in normal cells. The quantity required to achieve the beneficial effects of the present invention depends upon the antagonist compound employed, the chemotherapeutic agent employed and the quantity of such agent employed.

In general, the quantity of antagonist compound employed is from 2 to 75 mg/kg of animal to which the antagonist compound is administered. The present invention is able to achieve an enhanced chemotherapeutic effect on cancer cells while, at the same time, also protecting normal cells from damage by the chemotherapeutic agent in a wide variety of circumstances where traditional chemotherapy leads to damage of normal cells or tissues not involved in the disease process. Examples of the adverse effects on normal cells which result in traditional chemotherapy include:
(a) the killing of, or damage to, bone marrow cells,
(b) the killing of, or damage to, normal cells lining the gastrointestinal tract,
(c) the killing of, or damage to, normal heart muscle cells by anti-cancer drugs, such as doxorubicin (Adriamycin) and its analogues, including daunorubicin and epirubicin, and the non-related but cross-reactive compound, mitoxantrone, and
(d) damage to normal tissue, particularly in the kidneys by cisplatinum.

In cancer-bearing animals, DPPE treatment alone demonstrates little, if any, in-vivo effect on tumor growth. However, when combined with known anti-cancer drugs, a marked synergistic action is observed whereby tumors are inhibited or killed. This effect has led, for example, to marked regressions or cures in some animal cancers, such as sarcoma and melanoma.

As noted above, continued administration of the antagonist compound following administration of the chemotherapeutic agent, specifically up to 0.2 mg/kg of DPPE on a daily basis, at least ameliorates, and often eliminates, the side effects often associated with chemotherapy, including nausea, vomiting, anorexia and stomatitis, and preferably is effected herein, with the longer the period of administration, the more significant is the protection against the side effects. A daily dose of 0.1 to 5 mg/kg of such antagonist may be employed.

Such continued administration of antagonist component is most conveniently effected by intravenous administration, although oral administration may be feasible and, in some cases, more desirable from the standpoint of patient acceptance and of decreasing the load on the medical facility.

While the applicant does not wish to be bound by any theory to explain the beneficial effects achieved by the present invention, the following theory is proposed. The compound administered to the patient is a specific antagonist of histamine binding at a newly-discovered novel intracellular receptor (H_{IC}) (see, for example, "Histamine is an Intracellular Messenger Mediating Platelet Aggregation" by Saxena et al, Science, Vol 243, pg. 1596-1599). Intracellular histamine normally functions through this receptor to mediate or modulate many important cell functions, including cell proliferation, immune responses and platelet aggregation.

Protection of the normal cells is achieved through antagonism of histamine at H_{IC} by the antagonist. Such antagonism results in a temporary complete shut-down of cell division, so that normal cells are not susceptible to DNA damage in the presence of the chemotherapeutic agent(s), which preferentially attack dividing cells. In this way, for example, DPPE is effective to block therapy-associated toxicity of normal bone marrow stem cells.

In addition, the antagonism results in an increase in the levels of prostaglandins (natural substances which are known to protect tissues from various injurious agents) in the tissue. For example, DPPE treatment results in an increase by 500% in prostaglandin (PG)I₂, a protective prostaglandin, in the gut. Through this mechanism, DPPE is known to completely block ulcer formation in the presence of noxious agents, such as alcohol and cysteamine (see US-A-4,829,068).

The antagonist further effects a potent blockage of the degranulation of tissue mast cells, whose granular contents, including histamine itself, have been linked to tissue damage and severe systemic side effects. Certain anti-cancer drugs, such as adriamycin, cause significant mast cell degranulation, an effect which has been related to cardiotoxic effects.

As with bone marrow cells, the treatment of normal proliferating lymphocytes (immune cells), according to the invention, results in a dose-dependent blocking of DNA synthesis and a shut-down of these cells without causing cytotoxicity. The antagonist has an effect on both T-lymphocytes and B-lymphocytes in the immune system. For example, DPPE is able to completely antagonize proliferation of T-lymphocytes in the presence of Concanavalin A, a potent mitogen and plant lectin. DPPE also blocks the stimulation of antibody formation by the mediator interleukin-2 in certain B cells, resulting is a decrease in antibody formation.

In contrast to its cytoprotective effect on normal cells and tissue in vivo, as described herein, DPPE treatment damages and/or kills malignant cells in vitro, as described in US-A- No. 4,803,227, or those which are virally infected.

### EXAMPLES

### Example I

This Example illustrates in vivo augmentation by DPPE of adriamycin anti-tumor activity in a murine sarcoma model.

C-3 fibrosarcoma cells (3 x 10⁵) were injected into the left gluteal region of C3H mice on day 0. On day 1, the mice were provided with treatment by a combination of DPPE and adriamycin, administered intraperitoneally. The DPPE was administered 60 minutes prior to administration of the adriamycin. Mice also were administered with saline, DPPE alone and adriamycin alone.

Animals in the experiments (n=12) were followed for 60 days. At the end of the experimental period, those animals free of palpable tumors were considered cured.

The results obtained are set forth in the following Table I:

**TABLE I**

| **Treatment** | **Number of Rats Tumor-Free (n=12)** |
|---|---|
| Saline | 1 |
| Adriamycin (2mg/kg) | 0 |
| DPPE (50mg/kg) | 0 |
| DPPE (2mg/kg) / Adriamycin (2mg/kg) | 1 |
| DPPE (25mg/kg) / Adriamycin (2mg/kg) | 3 |
| DPPE (50mg/kg) / Adriamycin (2mg/kg) | 7 |

It will be seen from the results set forth in the above Table I, that, when adriamycin and DPPE alone are administered, no effect was obtained whereas when increasing quantities of DPPE were employed in combination with adriamycin, an increased anti-tumor activity was observed, such that, at the highest dose of DPPE tested (50mg/kg), 7 out of the 12 animals were cured.

### Example II

This Example illustrates protection of bone marrow progenitors by DPPE in mice treated with a lethal dose of 5FU and adriamycin.

Mice of the strain C57B1 were administered a lethal dose (7.5mg) of 5FU (5-fluorouracil), DPPE (100mg/kg) or a combination of a lethal dose of 5FU and DPPE (100mg/kg or 4mg/kg) and the results were compared with a control group to which saline only was administered. The DPPE and saline were administered 90 minutes prior to the 5FU. Bone marrow cell counts were made at 24 hours and 48 hours post administration.

The results obtained are set forth in the following Table IIA:

**TABLE IIA**

| Treatment | CFU-C/10⁴ cells ⁽¹⁾ | | R.C.S.⁽²⁾ | |
|---|---|---|---|---|
| | 24h | 48h | 24h | 48h |
| Saline | 38.3 | 40.3 | 1.0 | 1.0 |
| 5FU | 0.2 | 0.09 | 0.006 | 0.002 |
| DPPE | 36.7 | 38.6 | 0.96 | 0.96 |
| DPPE (100mg/kg) + 5FU | 35.7 | 37.3 | 0.93 | 0.93 |
| DPPE (4mg/kg) + 5FU | 38.3 | 33.3 | 1.0 | 0.83 |

| | | | | |
|---|---|---|---|---|
| Note: ⁽¹⁾ No. of bone marrow colonies. CFU-C = colony-forming units in culture | | | | |
| ⁽²⁾ Relative Cell Survival | | | | |

Experiments paralleling those described above with 5FU were carried out employing a lethal dose of adriamycin (20mg/kg). The results obtained from these experiments are set forth in the following Table IIB:

**TABLE IIB**

| Treatment | CFU-C/10⁴ cells | | R.C.S. | |
|---|---|---|---|---|
| | 24h | 48h | 24h | 48h |
| Saline | 43.7 | 42.7 | 1.0 | 1.0 |
| Adriamycin | 1.8 | 0.65 | 0.04 | 0.006 |
| DPPE (4mg/kg) | 42.3 | 41.3 | 0.97 | 0.97 |
| DPPE (4mg/kg) / Adriamycin | 39.7 | 39.0 | 0.91 | 0.91 |
| DPPE (100mg/kg) / Adriamycin | 43.3 | 41.3 | 0.99 | 0.97 |

As may be seen from the results set forth in the above Tables IIA and IIB, the administration of the DPPE along with the 5FU or adriamycin provided almost complete protection for bone marrow progenitors from the lethal effects of the 5FU or adriamycin.

### EXAMPLE III

This Example illustrates in vivo augmentation of BCNU anti-tumor activity in a B16 melanoma lung metastasis model.

5 x 10⁴ B16 melanoma cells were injected intravenously into the tail vein of C57Bl mice at day 0. The mice were treated with either saline, 32mg/kg of DPPE, 1mg of BCNU or a combination of 32mg/kg of DPPE and 1mg of BCNU, by intraperitoneal injection on day 1. The DPPE was administered 60 minutes before the BCNU.

In each group, six of the twelve animals were sacrificed at day 14 and lungs were removed for determination of metastasis. The remaining six animals were followed to death. The numbers and size of the lung metastases were determined by visual or microscope count.

The results obtained are set forth in the following Table III:

**TABLE III**

| Treatment | Numbers and (% control) of lung metastases | Size of lung metastases (1) | Median Survival (days) |
|---|---|---|---|
| Saline | 241 - | All Macro | 19 |
| DPPE | 219 (91%) | All Macro | 21 |
| BCNU | 144 (60%) | All Macro | 24 |
| DPPE/BCNU | 58 (27%) | All Micro | 32 |

| | | | |
|---|---|---|---|
| (1) Macro means visually determined. Micro means microscopically only. | | | |

As may be seen from the results in Table III, the inhibitory effect of BCNU on the lung tumors was significantly increased by the additional presence of DPPE, which itself alone had a marginal effect.

### EXAMPLE IV:

This Example illustrates in vivo augmentation of daunorubicin anti-tumor activity in a B16 melanoma lung metastasis model.

The procedure of Example III was repeated employing daunorubicin in place of BCNV. Groups of six mice were injected with B16f10 melanoma cells and 24 hours later received saline, 4 mg/kg of DPPE alone, a non-lethal dose of daunorubicin alone (12.5 mg/kg) or DPPE (4, 25 or 50 mg/kg) one hour prior to daunorubicin (12.5 mg/kg). All animals were followed to death or for 60-days post injection, and sacrificed for lung metastases.

The results obtained are set forth in the following Table IV:

**TABLE IV**

| Treatment Group | Median Survival (days) | No. Cures (n=6) |
|---|---|---|
| Saline | 17 | 0 |
| Daunorubicin (12.5 mg/kg) | 25 | 0 |
| DPPE (4 mg/kg) + Daunorubicin (12.5 mg/kg) | 29 | 2 |
| DPPE (25 mg/kg) + Daunorubicin (12.5 mg/kg) | 60⁺ | 4 |
| DPPE (50 mg/kg) + Daunorubicin (12.5 mg/kg) | 60⁺ | 4 |

As may be seen from the results of Table IV, the inhibitory effect of daunorubicin or lung tumors was enhanced by the presence of DPPE.

### EXAMPLE V:

This Example shows in vivo host cytoprotection from a lethal dose of adriamycin.

Saline or DPPE (2 mg/kg) were administered to DBA/2 mice 1 hour (n=12) or 15 minutes (n=6) prior to administration of 15 mg/kg of adriamycin. The number of survivors after 30 days was determined. The results are set forth in the following Table V:

**TABLE V**

| Treatment | Number of Survivors |
|---|---|
| Saline | 4/12 (33%) |
| DPPE | 13/18 (72%) |

As may be seen from Table V, the administration of DPPE provided in vivo host cytoprotection from the lethal dose of adriamycin.

### EXAMPLE VI:

This Example illustrates the effect of DPPE on thymidine incorporation into lymphocyte DNA.

Spleen cells from BALB/C mice were stimulated with Concanavalin A (5 µg/ml) for thymidine incorporation. The cells then were treated with varying does of DPPE and the level of thymidine incorporated was determined. The results were plotted graphically and appear as Figure 1. As may be seen from this Figure, at a dosage level of 25 µM, DPPE completely blocks thymidine incorporation into DNA but does not adversely affect cell survival. Thus, the DPPE treatment puts normal proliferating lymphocytes into a state of growth arrest without causing cytotoxicity.

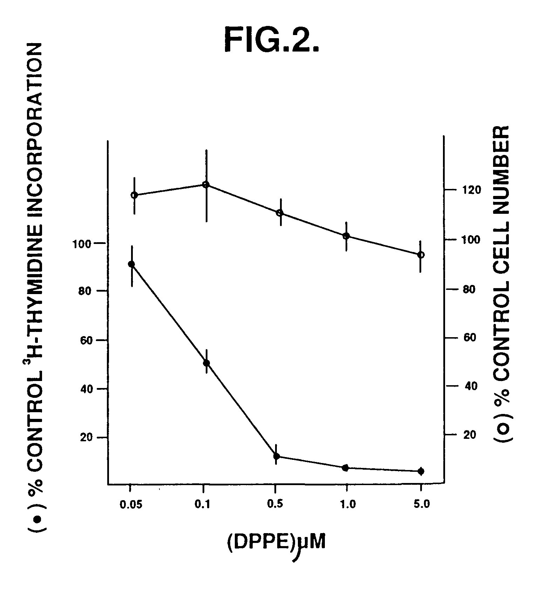

The experiment was repeated using 2.5 µg/ml of Concanavalin A in place of 5 µg/ml and 2% fetal calf serum in place of 10%. The results obtained are illustrated in Figure 2. At concentrations of DPPE which inhibited DNA synthesis (5 µm), no significant cytotoxicity was observed.

### EXAMPLE VII:

This Example also illustrates the effect of DPPE on thymidine incorporation into lymphocyte DNA.

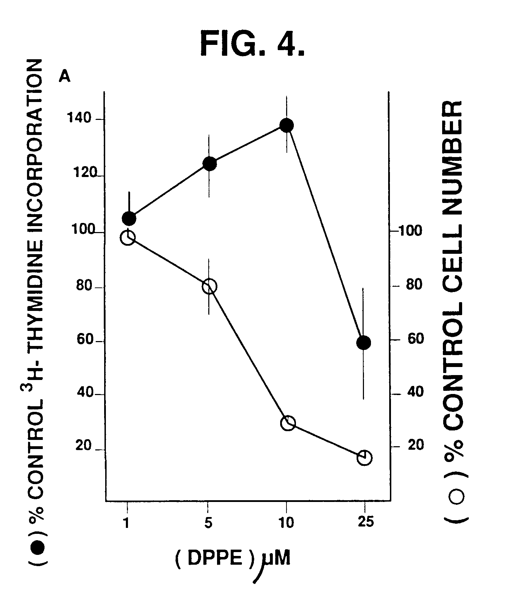

The experiment of Example VI was repeated employing virally-infected non-senescing transformed spleen-derived lymphocytes (S-10) also of BALB/C origin with 0.25 nM of ³H-thymidine added. The results were plotted graphically and appear as Figures 3 and 4 respectively. As may be seen therein, in contrast to Figures 1 and 2, 25 µM of DPPE caused approximately 50% cytotoxicity to the virally-infected cells. When adjusted for cell number, thymidine incorporation increased at cytotoxic concentrations of DPPE (10 to 25 µM).

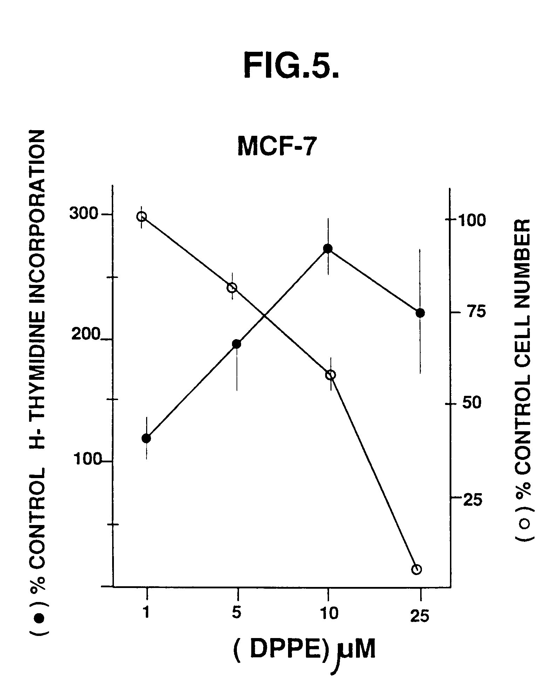

The experiment was again repeated using human breast cancer cell (MCF-7) with 0.25 nM of thymidine added. The results were plotted graphically and appear as Figure 5. Analogous results can be seen to those observed with the S-10 cells.

### Example VIII:

A clinical study was carried out with 14 patients with advanced cancer.
(a) DPPE alone was administered to patients to determine a safe dose range in humans. The highest non-toxic dosage was found to be 4 mg/kg given intravenously over a one-hour period. At 6 mg/kg over one hour, CNS toxicity (as manifested by any or all of muscle twitching, a drop of 1 to 2°C in body core temperature, auditory hyperacusis or hallucination, choreoathetosis, cerebellar ataxia and projectile vomiting) was dose limiting. However, significant toxicity was absent when 6 mg/kg was administered IV over 2 hours, suggesting that peak serum level determines CNS toxicity. When the dose is converted to mg/m², threshold for CNS toxicity occurs at the same dose previously observed in preclinical toxicology (ip route) studies in mice (240 mg/M²).
(b) DPPE at a daily dose of 0.2 mg/kg, given as an iv infusion over 24 to 72 hours was found to be entirely without clinical side effects, with the possible exception of constipation in occasional patients, and not to cause any significant changes in biochemistries or blood counts. This dose of DPPE also has been determined to potently prevent, or ameliorate by over 90%, nausea, vomiting, anorexia and stomatitis in 6/6 patients treated with Adriamycin (60 mg/m²). GI protection was most pronounced when DPPE was given at a dose of 0.2 mg/kg daily for 72 hours by IV infusion.
(c) Higher single iv doses of DPPE (1, 2, 4 mg/kg) given over 1 hour also appear to be significantly antiemetic against Adriamycin, although some patients experienced nausea, or transient anorexia, at 4 mg/kg of DPPE alone. At doses of 1 to 6 mg/kg iv over 1 hour, DPPE alone also was found to cause a transient decrease (20 to 30%) in neutrophil counts in 4/6 patients, with complete recovery by day 5 to 7. No significant effect of DPPE alone on platelets, hemoglobin or biochemistry has been observed.
(d) Using 0.2 mg/kg of DPPE as a daily dose, increased duration of treatment improved the therapeutic benefit to prevent nausea, vomiting, anorexia and a drop in nadir white counts, but not alopecia, caused by Adriamycin at a dosage level of 60 mg/m². A 24-hour DPPE infusion was an effective anti-emetic therapy in the first 24 to 48 hours following Adriamycin administration, but many patients then experienced delayed nausea, vomiting and/or anorexia at 72 or 96 hours after Adriamycin administration. However, when given as a 72-hour infusion, DPPE was observed to block completely all acute and delayed gastrointestinal side effects of Adriamycin in four patients, each of which had received two such treatments. In addition, two additional patients experienced only one minor episode of nausea and/or vomiting in the first 24 hours following Adriamycin administration, but then were well without any need for antiemetics. The 72-hour infusion of low dose DPPE also prevented mouth ulceration in one patient who had previously experienced this symptom during all previous non-Adriamycin chemotherapy and DPPE/Adriamycin given in shorter schedules.
(e) As compared to other regimens of DPPE/Adriamycin, nadir polymorphonuclear WBC counts at 14 days appear to be highest in patients who received 0.2 mg/kg DPPE for 72 hours (1,285 ± 385; mean ± S.E.M.). Platelet counts have been uniformly above 150,000/mm³ at Day 14.
(f) In fifteen evaluable patients, five major responses, two breast, one lymphoma and one medullary carcinoma of thyroid, have been documented.

## Claims

1. The use of an antagonist specific for intracellular histamine for the manufacture of a medicament for the treatment of cancer cells in a living animal (a) by administration to said animal in an amount sufficient to inhibit the binding of intracellular histamine in normal cells prior to administration of at least one chemotherapeutic agent for the cancer cells in an amount toxic to said cancer cells, to enhance the toxic effect on said cancer cells of said at least one chemotherapeutic agent while inhibiting any adverse effect of said at least one chemotherapeutic agent on said normal cells and/or (b) by administration to said animal in an amount sufficient to inhibit binding of intracellular histamine in normal cells over a period of at least twenty-four hours subsequent to administration of at least one chemotherapeutic agent for the cancer cells in an amount toxic to said cancer cells, to at least ameliorate the side effects of the administration of said at least one chemotherapeutic agent.

2. The use as claimed in Claim 1 of an antagonist specific for intracellular histamine for the manufacture of a medicament for the treatment of cancer cells in a living animal by administration to said animal in an amount sufficient to inhibit the binding of intracellular histamine in normal cells prior to administration of at least one chemotherapeutic agent for the cancer cells in an amount toxic to said cancer cells, to enhance the toxic effect on said cancer cells of said at least one chemotherapeutic agent while inhibiting any adverse effect of said at least one chemotherapeutic agent on said normal cells.

3. A use as claimed in Claim 2, wherein said antagonist specific for intracellular histamine is a diphenylmethane of the formula: 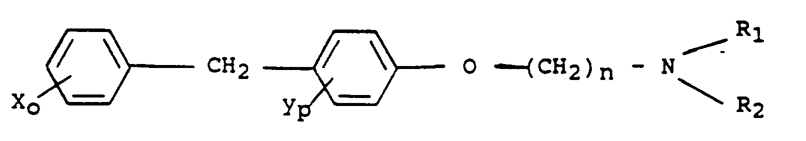 wherein X and Y are each chlorine or bromine, o and p are 0 or 1, R₁ and R₂ are each alkyl groups containing 1 to 3 carbon atoms or are joined together to form a hetero-ring with the nitrogen atom and n is 1, 2 or 3, or a pharmaceutically-acceptable salt thereof.

4. A use as claimed in Claim 3, wherein the group 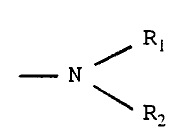 is a diethylamino group or a morpholino group.

5. A use as claimed in Claim 4, wherein the group 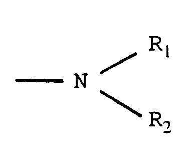 is a diethylamino group, n is 2, and o and p are each 0.

6. A use as claimed in Claim 5, wherein the diphenylmethane is in the form of a hydrochloride salt.

7. A use as claimed in any one of the preceding claims, wherein said medicament is in a form suitable for administering in an amount of from 2 to 75 mg/kg of animal.

8. A use as claimed in Claim 7, wherein the medicament is for protecting normal bone marrow cells from the adverse effects of said at least one chemotherapeutic agent.

9. A use as claimed in Claim 7, wherein the medicament is for protecting normal heart muscle cells from the adverse effects of said at least one chemotherapeutic agent.

10. A use as claimed in Claim 7, wherein the medicament is for protecting normal cells lining the gastrointestinal tract from the adverse effects of said at least one chemotherapeutic agent.

11. The use as claimed in Claim 1 of an antagonist specific for intracellular histamine for the manufacture of a medicament for the treatment of cancer cells in a living animal by administration to said animal in an amount sufficient to inhibit the binding of intracellular histamine in normal cells over a period of at least twenty-four hours subsequent to administration of at least one chemotherapeutic agent for the cancer cells in an amount toxic to said cancer cells, to at least ameliorate the side effects of the administration of said at least one chemotherapeutic agent.

12. A use as claimed in Claim 11, wherein said antagonist specific for intracellular histamine is a diphenylmethane of the formula: 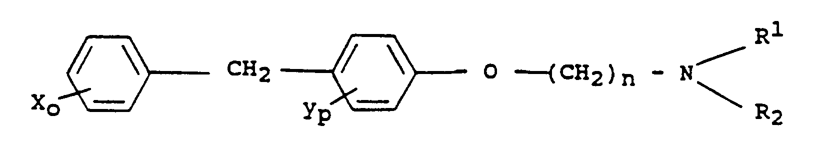 wherein X and Y are each chlorine or bromine, o and p are 0 or 1, R₁ and R₂ are each alkyl groups containing 1 to 3 carbon atoms or are joined together to form a hetero-ring with the nitrogen atom and n is 1, 2 or 3, or a pharmaceutically-acceptable salt thereof.

13. A use as claimed in Claim 12, wherein the group 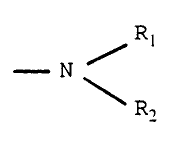 is a diethylamino group or a morpholino group.

14. A use as claimed in Claim 13, wherein the group 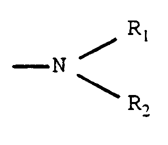 is a diethylamino group, n is 2, and o and p are each 0.

15. A use as claimed in Claim 14, wherein the diphenylmethane is in the form of a hydrochloride salt.

16. A use as claimed in any one of Claims 11 to 15, wherein said medicament is in a form suitable for administering at a daily dosage of up to 0.2 mg/kg.

17. A kit for the treatment of cancer cells in a living animal comprising:
(a) a first component consisting of an antagonist specific for intracellular histamine in a dosage amount sufficient to inhibit the binding of intracellular histamine in normal cells of the animal, and, separately,
(b) a second component consisting of at least one chemotherapeutic agent for the cancer cells in a dosage amount toxic to said cancer cells, and, separately:
(c) a third component consisting of an antagonist specific for intracellular histamine in a dosage amount sufficient to ameliorate the side effects of administration of the chemotherapeutic agent to the animal.

18. A kit as claimed in Claim 17, wherein said antagonist specific for intracellular histamine is as defined in any one of Claims 3 to 7 for component (a) and as defined in any one of Claims 12 to 16 for component (c).

## Patentansprüche

1. Verwendung eines für intrazelluläres Histamin spezifischen Antagonisten zur Herstellung eines Arzneimittels zur Behandlung von Krebszellen in einem lebenden Tier, durch
(a) Verabreichen desselben an das Tier in einer ausreichenden Menge, um das Binden von intrazellulärem Histamin in normalen Zellen vor einem Verabreichen von mindestens einem chemotherapeutischen Mittel für die Krebszellen in einer für die Krebszellen toxischen Menge zu hemmen, um die toxische Wirkung des mindestens einen chemotherapeutischen Mittels auf die Krebszellen zu verstärken, wobei jegliche abträgliche Wirkung des mindestens einen chemotherapeutischen Mittels auf die normalen Zellen gehemmt wird, und/oder
(b) Verabreichen desselben an das Tier in einer ausreichenden Menge, um ein Binden von intrazellulärem Histamin in normalen Zellen für eine Zeitdauer von mindestens 24 Stunden nach Verabreichen von mindestens einem chemotherapeutischen Mittel für die Krebszellen in einer für die Krebszellen toxischen Menge zu hemmen, um die Nebenwirkungen des Verabreichens des mindestens einen chemotherapeutischen Mittels zumindest abzuschwächen.

2. Verwendung eines für intrazelluläres Histamin spezifischen Antagonisten für die Herstellung eines Arzneimittels für die Behandlung von Krebszellen in einem lebenden Tier nach Anspruch 1 durch Verabreichen desselben an das Tier in einer ausreichenden Menge, um das Binden von intrazellulärem Histamin in normalen Zellen vor einem Verabreichen von mindestens einem chemotherapeutischen Mittel für die Krebszellen in einer für die. Krebszellen toxischen Menge Zu hemmen, um die toxische Wirkung des mindestens einen chemotherapeutischen Mittels auf die Krebszellen zu verstärken, wobei jegliche abträgliche Wirkung des mindestens einen chemotherapeutischen Mittels auf die normalen Zellen gehemmt wird.

3. Verwendung nach Anspruch 2,
wobei der für intrazelluläres Histamin spezifische Antagonist ein Diphenylmethan der Formel: 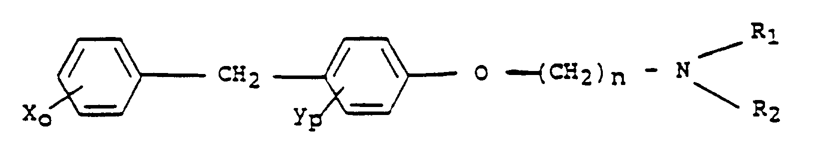 ist,
worin X und Y jeweils Chlor oder Brom ist, o und p 0 oder 1 sind, R₁ und R₂ jeweils Alkylgruppen mit 1 bis 3 Kohlenstoffatomen sind oder miteinander unter Bildung eines Heterozyklus mit dem Stickstoffatom verbunden sind und n 1, 2 oder 3 ist, oder ein pharmazeutisch akzeptables Salz desselben.

4. Verwendung nach Anspruch 3,
wobei die Gruppe 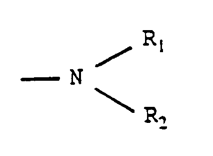 eine Diethylamin-Gruppe oder eine Morpholin-Gruppe ist.

5. Verwendung nach Anspruch 4,
wobei die Gruppe 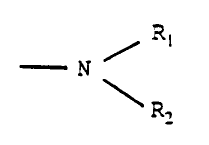 eine Diethylamin-Gruppe ist, n 2 ist und o und p jeweils 0 sind.

6. Verwendung nach Anspruch 5,
wobei das Diphenylmethan in Form eines Hydrochlorid-Salzes vorliegt.

7. Verwendung nach irgendeinem der vorangegangenen Ansprüche,
wobei das Arzneimittel in einer Form vorliegt, die geeignet ist, eine Menge von 2 bis 75 mg/kg Tier zu verabreichen.

8. Verwendung nach Anspruch 7,
wobei das Arzneimittel zum Schutz normaler Knochenmarkszellen vor den abträglichen Wirkungen des mindestens einen chemotherapeutischen Mittels dient.

9. Verwendung nach Anspruch 7,
wobei das Arzneimittel zum Schutz normaler Herzmuskelzellen vor den abträglichen Wirkungen des mindestens einen chemotherapeutischen Mittels dient.

10. Verwendung nach Anspruch 7,
wobei das Arzneimittel zum Schutz von normalen, den Gastrointestinaltrakt auskleidenden Zellen vor den abträglichen Wirkungen des mindestens einen chemotherapeutischen Mittels dient.

11. Verwendung eines für intrazelluläres Histamin spezifischen Antagonisten für die Herstellung eines Arzneimittels für die Behandlung von Krebszellen in einem lebenden Tier nach Anspruch 1 durch Verabreichen desselben an das Tier in einer ausreichenden Menge, um das Binden von intrazellulärem Histamin in normalen Zellen für eine Zeitdauer von mindestens 24 Stunden nach einem Verabreichen von mindestens einem chemotherapeutischen Mittel für die Krebszellen in einer für die Krebszellen toxischen Menge zu hemmen, um die Nebenwirkungen des Verabreichens des mindestens einen chemotherapeutischen Mittels zumindest abzuschwächen.

12. Verwendung nach Anspruch 11,
wobei der für intrazelluläres Histamin spezifische Antagonist ein Diphenylmethan der Formel: 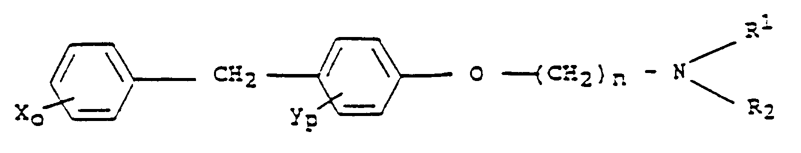 ist, worin X und Y jeweils Chlor oder Brom ist, o und p 0 oder 1 sind, R₁ und R₂ jeweils Alkylgruppen mit 1 bis 3 Kohlenstoffatomen sind oder miteinander unter Bildung eines Heterozyklus mit dem Stickstoffatom verbunden sind und n 1, 2 oder 3 ist, oder ein pharmazeutisch akzeptables Salz desselben.

13. Verwendung nach Anspruch 12,
wobei die Gruppe 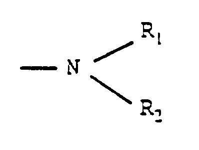 eine Diethylamin-Gruppe oder eine Morpholin-Gruppe ist.

14. Verwendung nach Anspruch 13,
wobei die Gruppe 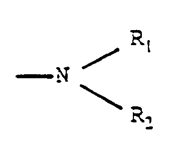 eine Diethylamin-Gruppe ist, n 2 ist und o und p jeweils 0 sind.

15. Verwendung nach Anspruch 14,
wobei das Diphenylmethan in Form eines Hydrochlorid-Salzes vorliegt.

16. Verwendung nach irgendeinem der Ansprüche 11 bis 15,
wobei das Arzneimittel in einer Form vorliegt, die geeignet ist, eine tägliche Dosis von bis zu 0,2 mg/kg zu verabreichen.

17. Kit zur Behandlung von Krebszellen in einem lebenden Tier, umfassend
(a) einen ersten Bestandteil, bestehend aus einem für intrazelluläres Histamin spezifischen Antagonisten in einer ausreichenden Verabreichungsmenge, um das Binden von intrazellulärem Histamin in normalen Zellen des Tiers zu hemmen, und davon getrennt
(b) einen zweiten Bestandteil, bestehend aus mindestens einem chemotherapeutischen Mittel für die Krebszellen in einer für die Krebszellen toxischen Verabreichungsmenge, und davon getrennt
(c) einen dritten Bestandteil, bestehend aus einem für intrazelluläres Histamin spezifischen Antagonisten in einer ausreichenden Verabreichungsmenge, um die Nebenwirkungen eines Verabreichens des chemotherapeutischen Mittels an das Tier abzuschwächen.

18. Kit nach Anspruch 17,
wobei der für intrazelluläres Histamin spezifische Antagonist für den Bestandteil (a) wie in irgendeinem der Ansprüche 3 bis 7 beschrieben und für den Bestandteil (c) wie in irgendeinem der Ansprüche 12 bis 16 beschrieben ist.

## Revendications

1. Utilisation d'un antagoniste spécifique de l'histamine intracellulaire pour la préparation d'un médicament destiné au traitement de cellules cancéreuses chez un animal vivant (a) par administration audit animal en une quantité suffisante pour inhiber la fixation d'histamine intracellulaire dans des cellules normales avant l'administration d'au moins un agent chimiothérapeutique destiné aux cellules cancéreuses en une quantité toxique pour lesdites cellules cancéreuses, pour amplifier l'effet toxique sur lesdites cellules cancéreuses dudit agent chimiothérapeutique au nombre minimal de un, tout en inhibant tout effet adverse éventuel dudit agent chimiothérapeutique au nombre minimal de un sur lesdites cellules normales et/ou (b) par administration audit animal en une quantité suffisante pour inhiber la fixation d'histamine intracellulaire dans des cellules normales sur une durée d'au moins vingt quatre heures après l'administration d'au moins un agent chimiothérapeutique destiné aux cellules cancéreuses en une quantité toxique pour lesdites cellules cancéreuses, pour au moins améliorer les effets secondaires de l'administration dudit agent chimiothérapeutique au nombre minimal de un.

2. Utilisation selon la revendication 1 d'un antagoniste spécifique de l'histamine intracellulaire pour la préparation d'un médicament destiné au traitement de cellules cancéreuses chez un animal vivant, par administration audit animal en une quantité suffisante pour inhiber la fixation d'histamine intracellulaire dans des cellules normales avant l'administration d'au moins un agent chimiothérapeutique destiné aux cellules cancéreuses en une quantité toxique pour lesdites cellules cancéreuses, pour amplifier l'effet toxique sur lesdites cellules cancéreuses dudit agent chimiothérapeutique au nombre minimal de un, tout en inhibant tout effet adverse éventuel dudit agent chimiothérapeutique au nombre minimal de un sur lesdites cellules normales.

3. Utilisation selon la revendication 2, dans laquelle ledit antagoniste spécifique de l'histamine intracellulaire est un diphénylméthane de formule : 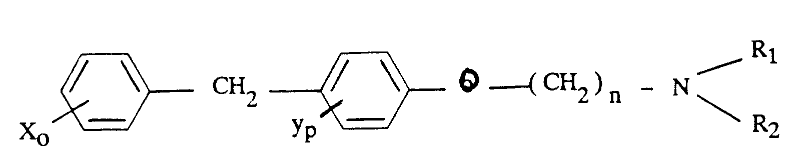 dans laquelle X et Y sont chacun un atome de chlore ou de brome, o et p sont égaux à 0 ou 1, R₁ et R₂ sont l'un et l'autre des groupements alkyles contenant 1 à 3 atomes de carbone ou sont réunis pour former un hétérocycle avec l'atome d'azote et n est égal à 1,2 ou 3,
ou un sel pharmaceutiquement acceptable de ce dernier.

4. Utilisation selon la revendication 3, dans laquelle le groupement 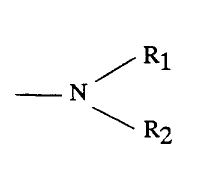 est un groupement diéthylamino ou un groupement morpholino.

5. Utilisation selon la revendication 4, dans laquelle le groupement 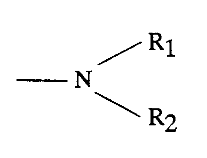 est un groupement diéthylamino, n est égal à 2 et o et p sont l'un et l'autre égaux à 0.

6. Utilisation selon la revendication 5, dans laquelle le diphénylméthane se présente sous la forme d'un sel de type chlorhydrate.

7. Utilisation selon l'une quelconque des revendications précédentes, dans laquelle ledit médicament se présente sous une forme convenant à l'administration en une quantité de 2 à 75 mg/kg d'animal.

8. Utilisation selon la revendication 7, dans laquelle le médicament est destiné à protéger les cellules normales de la moelle osseuse des effets adverses dudit agent chimiothérapeutique au nombre minimal de un.

9. Utilisation selon la revendication 7, dans laquelle le médicament est destiné à protéger les cellules normales myocardiques des effets adverses dudit agent chimiothérapeutique au nombre minimal de un.

10. Utilisation selon la revendication 7, dans laquelle le médicament est destiné à protéger les cellules normales tapissant le tractus gastro-intestinal des effets adverses dudit agent chimiothérapeutique au nombre minimal de un.

11. Utilisation selon la revendication 1 d'un antagoniste spécifique de l'histamine intracellulaire pour la préparation d'un médicament destiné au traitement de cellules cancéreuses chez un animal vivant par administration audit animal en une quantité suffisante pour inhiber la fixation d'histamine intracellulaire dans des cellules normales pendant une durée d'au moins vingt quatre heures après l'administration d'au moins un agent chimiothérapeutique destiné aux cellules cancéreuses en une quantité toxique pour lesdites cellules cancéreuses, pour au moins améliorer les effets secondaires de l'administration dudit agent chimiothérapeutique au nombre minimal de un.

12. Utilisation selon la revendication 11, dans laquelle ledit antagoniste spécifique de l'histamine intracellulaire est un diphénylméthane de formule : 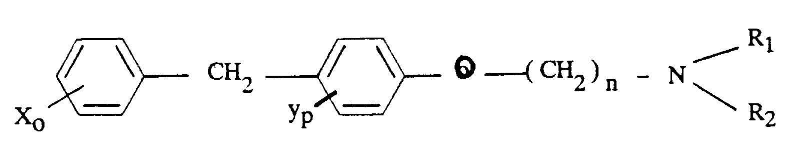 dans laquelle X et Y sont chacun un atome de chlore ou de brome, o et p sont égaux à 0 ou 1, R₁ et R₂ sont l'un et l'autre des groupements alkyles contenant 1 à 3 atomes de carbone ou sont réunis pour former un hétérocycle avec l'atome d'azote et n est égal à 1, 2 ou 3,
ou un sel pharmaceutiquement acceptable de ce dernier.

13. Utilisation selon la revendication 12, dans laquelle le groupement 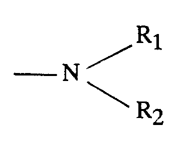 est un groupement diéthylamino ou un groupement morpholino.

14. Utilisation selon la revendication 13, dans laquelle le groupement 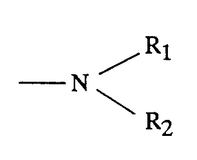 est un groupement diéthylamino, n est égal à 2 et o et p sont l'un et l'autre égaux à 0.

15. Utilisation selon la revendication 14, dans laquelle le diphénylméthane se présente sous la forme d'un sel de type chlorhydrate.

16. Utilisation selon l'une quelconque des revendications 11 à 15, dans laquelle ledit médicament se présente sous une forme convenant à l'administration en une posologie quotidienne maximale de 0,2 mg/kg.

17. Nécessaire de traitement de cellules cancéreuses chez un animal vivant, comprenant :
(a) un premier composant consistant en un antagoniste spécifique de l'histamine intracellulaire en une dose suffisante pour inhiber la fixation d'histamine intracellulaire dans les cellules normales de l'animal et, séparément,
(b) un second composant consistant en au moins un agent chimiothérapeutique destiné aux cellules cancéreuses en une dose toxique pour lesdites cellules cancéreuses et, séparément,
(c) un troisième composant consistant en un antagoniste spécifique de l'histamine intracellulaire en une dose suffisante pour améliorer les effets secondaires de l'administration de l'agent chimiothérapeutique à l'animal.

18. Nécessaire selon la revendication 17, dans lequel ledit antagoniste spécifique de l'histamine intracellulaire est tel que défini dans l'une quelconque des revendications 3 à 7 pour le composant (a) et tel que défini dans l'une quelconque des revendications 12 à 16 pour le composant (c).
